# EUROPÄISCHE PATENTSCHRIFT

(11) **EP 3 818 922 B1**
(45) Veröffentlichungstag und Bekanntmachung des Hinweises auf die Patenterteilung: **21.02.2024**
(21) Anmeldenummer: 20191125.2
(22) Anmeldetag: 14.08.2020
(51) Int. Cl.: A61B 1/12, F21V 29/71, H01L 23/40, H05K 1/02

(54) **KÜHLVORRICHTUNG FÜR EIN ENDOSKOP ODER EXOSKOP**
COOLING DEVICE FOR AN ENDOSCOPE OR EXOSCOPE
DISPOSITIF DE REFROIDISSEMENT POUR UN ENDOSCOPE OU UN EXOSCOPE

(30) Priorität: 05.11.2019 DE 102019129815
(43) Veröffentlichungstag der Anmeldung: 12.05.2021
(73) Patentinhaber: Karl Storz SE & Co. KG, 78532 Tuttlingen (DE)
(72) Erfinder: Geafer, Lawrence, 78532 Tuttlingen (DE); Heni, Andreas, 78532 Tuttlingen (DE); Kupferschmid, Markus, 78532 Tuttlingen (DE)
(74) Vertreter: Witte, Weller & Partner Patentanwälte mbB

(56) Entgegenhaltungen:
- JP-A- 2012 050 704
- US-A1- 2009 315 986
- US-A1- 2011 237 886
- US-A1- 2016 278 620

## Beschreibung

Die vorliegender Erfindung betrifft eine Kühlvorrichtung für ein Endoskop oder Exoskop sowie ein System mit einer Kühlvorrichtung.

Endoskope und Exoskope führen die durch Lichtverluste und elektrische Verluste entstehende Wärme passiv über ihre Oberfläche ab. Das führt dazu, dass sich diese Instrumente als Ganzes erwärmen. Entsprechende konstruktive Bemühungen zum Abführen der Wärme haben ihren Schwerpunkt darauf gerichtet, dass die im Betrieb erreichten Oberflächentemperaturen unterhalb der in der DIN EN 60601-1 Norm definierten Maximaltemperaturen liegen.

US 2016/0278620 A1 offenbart ein Bildaufnahmegerät mit einer Bildaufnahmevorrichtung, auf der ein Lichtempfangsabschnitt ausgebildet ist, einem Wärmeübertragungselement, wobei das Wärmeübertragungselement eine Verdrahtungsplatte, einem mit der Bildaufnahmevorrichtung verbundenen Verbindungsabschnitt, einem sich von dem Verbindungsabschnitt erstreckenden Biegeabschnitt und einem Gehäuse aus Metall, dessen Innenfläche mit einem Teil des festen Abschnitts des auf einer Innenseite untergebrachten Wärmeübertragungselements in Kontakt steht.

US 2009/0315986 A1 offenbart ein Endoskop aufweisend ein Bildaufnahmeelement, ein Bildaufnahmeelement-Gehäuserohr, in dem das Bildaufnahmeelement untergebracht ist, ein Wärmeübertragungselement, das in Kontakt mit dem Bildaufnahmeelement und dem Bildaufnahmeelement-Gehäuserohr steht, und ein Harz, das das Innere des Bildaufnahmeelement-Gehäuserohrs abdichtet und in Kontakt mit dem Bildaufnahmeelement steht. Das Wärmeübertragungselement hat eine höhere Wärmeleitfähigkeit als das Harz.

Es ist eine Aufgabe der vorliegenden Erfindung eine verbesserte Kühlvorrichtung für ein Endoskop oder Exoskop aufzuzeigen, die mit einem geringen konstruktiven Aufwand eine ausreichende Kühlung bereitstellt.

Die Aufgabe wird nach einem Aspekt gelöst durch eine Kühlvorrichtung für ein Endoskop oder ein Exoskop nach Anspruch 1.

Die Erfinder haben erkannt, dass die Erfüllung der Norm DIN EN 60601-1 natürlich einen wichtigen Aspekt darstellt, dass jedoch die Erwärmung der elektrischen Bauteile, insbesondere der Bildsensoren, einen limitierenden Faktor bei der Bildqualität darstellt. Diese ist abhängig von der Betriebstemperatur, denn je wärmer sie werden, desto mehr Dunkelstromrauschen entsteht. In den Datenblättern sind hierzu maximale Temperaturen angegeben, bei denen eine "gute" Bildqualität noch garantiert wird.

Gerade bei Exoskopen, insbesondere wenn sie nicht in der Hand gehalten werden, sind die Temperaturen, die für eine noch hinreichende Kühlung des gesamten Systems erlaubt sind, gewöhnlich höher, als die erlaubten Oberflächentemperaturen für Instrumente laut der Norm DIN EN 60601-1, was überraschenderweise eine passive Wärmeabfuhr (durch Wärmeleitung und freie Konvektion) der Bildsensoren ermöglicht. Für gewöhnlich sind die Exoskope auch leistungsfähiger, produzieren also mehr Verlustleistung als Endoskope, was dazu führt, dass die höheren Oberflächentemperaturen auch erreicht werden. Dies hat es im Stand der Technik jedoch verhindert, die verbauten Bildsensoren passiv zu kühlen, da diese als Wärmequelle immer wärmer als die sie umgebenden Bauteile sein müssen, um die Verlustwärme an sie abgeben zu können.

Eine der Besonderheiten der vorgeschlagenen Kühlvorrichtung ist, dass die mechanische Kopplung der Wärmequelle relativ zum Gehäuse von der thermischen Kopplung der Wärmequelle an das Gehäuse getrennt ist. Dies wird zum einen dadurch erzielt, dass das Trägerelement, das Wärmesperrelement und das Gehäuse eine mechanisch steife Einheit bilden, wobei das Wärmesperrelement eine geringe Wärmeleitfähigkeit hat. Zum anderen wird dies dadurch erzielt, dass das Wärmeelement, das zwischen dem Gehäuse und dem Trägerelement angeordnet ist, eine hohe Wärmeleitfähigkeit hat.

Der Begriff "steif" soll dahingehend verstanden werden, dass die Anordnung von Trägerelement, Wärmesperrelement und Gehäuse zumindest im Wesentlichen unverändert bleibt, wenn das Wärmeleitelement bei der Herstellung zwischen dem Träger Element und dem Gehäuse angeordnet wird. Ziel dieser Steifigkeit ist es, dass sowohl die zweite Oberfläche des Wärmeleitelements gegen die erste Oberfläche des Trägerelements gedrückt wird als auch die dritte Oberfläche des Wärmeleitelements in Richtung oder auf die vierte Oberfläche des Gehäuses gedrückt wird. Auf diese Weise liegen die jeweiligen Oberflächen fest gegeneinander an und erlauben einen guten Wärmefluss von der Wärmequelle über das Wärmeleitelement zum Gehäuse.

Die Begriffe "geringe Wärmeleitfähigkeit" und "hohe Wärmeleitfähigkeit" sind dahingehend zu verstehen, dass mittels der geringen Wärmeleitfähigkeit ein Wärmefluss über das Wärmesperrelement zum Gehäuse zumindest im Wesentlichen gering gehalten oder verhindert wird und dass mittels der hohen Wärmeleitfähigkeit ein Wärmefluss über das Wärmeleitelement zum Gehäuse gefördert werden soll. Ziel ist es dabei insbesondere, dass der gesamte Wärmefluss von der Wärmequelle zum Gehäuse zu mindestens 80 %, bevorzugt mindestens 85 %, besonders bevorzugt mindestens 90 % und insbesondere mindestens 95 % über das Wärmeleitelement erfolgt. Bei einigen Ausgestaltungen beträgt dieser Wert mindestens 97 %, bevorzugt mindestens 99 %, besonders bevorzugt 99,5 % und insbesondere 99,9 %.

Bei der Wahl der Materialien für das Wärmeleitelement und das Wärmesperrelement orientiert sich der Fachmann daran, welche Steifigkeit gewünscht ist und welcher Anteil des Wärmeflusses mindestens über das Wärmeleitelement geführt werden soll. Dabei kommt grundsätzlich eine große Anzahl von Materialkombinationen infrage, sofern dadurch der Aspekt der Trennung zwischen der mechanischen Kopplung und der thermischen Kopplung realisiert werden kann.

Dadurch, dass das Wärmeleitelement separat von Trägerelement und Gehäuse ausgebildet ist und die zweite Oberfläche in einem Winkel zur dritten Oberfläche steht, kann ein einfacher konstruktiver Aufbau erzielt werden, mit dem gleichzeitig eine gute Wärmeübertragung von der Wärmequelle auf das Gehäuse als Wärmesenke erzielt werden kann.

Bei einigen bevorzugten Ausgestaltungen wird das Wärmeleitelement beim Zusammenbau der Kühlvorrichtung zwischen das Trägerelement und das Gehäuse eingeschoben. Aufgrund des Winkels zwischen der zweiten und dritten Oberfläche, ähnlich einer schiefen Ebene, kommt das Wärmeleitelement zunächst in physikalischen Kontakt und beginnt dann, wenn es weiter eingeschoben wird, eine Kraft auf das Gehäuse und das Trägerelement auszuüben. Da diese Elemente jedoch steif sind und es sich in ihrer Position relativ zueinander zumindest im Wesentlichen nicht verändern, üben diese Elemente nun eine Gegenkraft auf das Wärmeleitelement aus. Dadurch wird die erste Oberfläche gegen die zweite Oberfläche gedrückt und die dritte Oberfläche in Richtung der oder gegen die vierte Oberfläche gedrückt. Daraus resultiert ein besonders guter Wärmefluss. Aufgrund des Winkels können Toleranzen innerhalb des Aufbaus kompensiert werden.

Bei einigen bevorzugten Ausgestaltungen steht die zweite Oberfläche und/oder dritte Oberfläche auch in einem Winkel zur vierten Oberfläche. Auf diese Weise kann die Kühlvorrichtung leicht an die jeweilige Anwendungssituation angepasst werden. Bei anderen bevorzugten Ausgestaltungen ist die erste Oberfläche parallel zur zweiten Oberfläche und/oder ist die dritte Oberfläche parallel zur vierten Oberfläche. Dies ermöglicht einen besonders guten Kontakt zwischen diesen zwei Oberflächenpaaren.

Bei einigen bevorzugten Ausführungsformen wird ein thermoelektrisches Kühlmodul eingesetzt, dass die Wärmequelle, insbesondere Bildsensoren, direkt kühlt. Dabei handelt es sich um eine Art Wärmepumpe, die einem Ort Wärme entzieht und an einen anderen Ort transportiert. Dafür muss Leistung erbracht werden, die in Form zusätzlicher Wärme abgeführt werden muss. In diesem Fall wird die zusätzliche Wärme passiv an das Gehäuse abgegeben, welches sich dann weiter erwärmt und mehr Wärme abgibt.

Grundsätzlich kann zusätzlich auch eine externe, aktive Kühlung verwendet werden. Ein Kühlmedium wie Wasser oder Luft wird mittels externer Pumpe durch Schläuche in das Endoskop oder Exoskop befördert, um die Bildsensoren zu kühlen. Dies erfordert ein weiteres, externes Gerät. Die vorgeschlagene Lösung kann allerdings auf dieses weitere externe Gerät verzichten. Auch müssen keine weiteren Leitungen neben dem Strom- und Datenkabel und dem Lichtleiterkabel verlegt werden, was als störend empfunden wird. Zudem entstehen keine Geräusche durch Fluidströmungen.

Grundsätzlich kann zusätzlich auch eine interne, aktive Kühlung verwendet werden. Ein in das Exoskop eingebauter Lüfter sorgt für eine Kühlung der Bildsensoren. Die externen Schläuche entfallen dabei. Die vorgeschlagene Lösung bietet demgegenüber aber auch hier Vorteile, da keine Geräusche durch die Kühlung entstehen. Außerdem können keine Partikel, insbesondere Staub, in das Endoskop oder Exoskop gelangen und sich daher nicht auf optischen Komponenten niederlassen.

Bei einer bevorzugten Ausführungsform befinden sich Bildsensoren als Wärmequelle, üblicherweise auf einer Platine, auf einem Bildsensorhalter als Trägerelement, über den Ausrichtung und Position der Bildsensoren eingestellt werden. Dieses besteht aus Aluminium, leitet die Wärme der Bildsensoren also effektiv an ein Kühlelement. Dieses ist zwischen Bildsensorhalter und einem Kühlkörper als Wärmesenke, der die Verlustwärme möglichst breit verteilt, positioniert. Der Bildsensorhalter berührt weder die umliegenden Teile noch den Kühlkörper, damit das Kühlmodul möglichst nur die Verlustwärme der Bildsensoren abführt. Auch ein etwaiger Rückfluss der Wärme vom Kühlkörper zum Bildsensorhalter über die Schrauben wird mit Unterlegscheiben aus schlecht wärmeleitfähigem Material unterbunden. Dies ist der Effektivität der Sensorkühlung geschuldet. Je weniger Wärmeleistung das Kühlmodul transportieren muss, desto niedriger können die Temperaturen auf der kalten Seite werden.

Das Wärmeleitelement ist dazu ausgebildet, zu seiner Positionierung zwischen das Trägerelement und das Gehäuse eingeschoben zu werden und dabei mit seiner zweiten Oberfläche auf die erste Oberfläche des Trägerelements aufgleitet.

Dies ist fertigungstechnisch gut zu realisieren und bewirkt einen guten Oberflächenkontakt zwischen der ersten oder zweiten Oberfläche.

Bei einer bevorzugten Ausgestaltung ist das Wärmeleitelement keilförmig ausgebildet.

Diese Ausgestaltung ist fertigungstechnisch gut zu realisieren und bewirkt einen guten Oberflächenkontakt zwischen der ersten oder zweiten Oberfläche.

Bei einer bevorzugten Ausgestaltung weist Wärmeleitelement ein Befestigungselement auf, mit dem das Wärmeleitelement an dem Trägerelement befestigt werden kann.

Diese Ausgestaltung stellt einerseits sicher, dass das Wärmeleitelement dauerhaft in einer definierten Position bleibt und sich nicht, insbesondere im Hinblick auf die stets auftretenden thermischen Veränderungen, verlagert. Bei dem Befestigungselement handelt es sich bevorzugt um eine Schraube, die in ein Gewinde im Trägerelement eingreift.

Bei einer bevorzugten Ausgestaltung weist das Befestigungselement ein flexibles Element auf, welches zwischen dem Wärmeleitelement und dem Trägerelement angeordnet ist und dafür ausgebildet ist, einen Druck zwischen dem Wärmeleitelement und dem Trägerelement zu verteilen und/oder zu reduzieren.

Diese Ausgestaltung ermöglicht einen besonders guten Sitz des Wärmeleitelements relativ zum Trägerelement, ohne dabei einen übermäßigen Druck auf das Trägerelement auszuüben.

Bei einer bevorzugten Ausgestaltung weist das Gehäuse einen Gehäusedeckel auf, der die Anordnung von Wärmequelle, Trägerelement und Wärmeleitelement überdeckt.

Diese Ausgestaltung ermöglicht es, während der Installation der Kühlvorrichtung Zugriff auf das Innere des Gehäuses zu haben und nach der Installation das Gehäuse zu verschließen. In Abhängigkeit von der jeweiligen Einbausituation kann der Gehäusedeckel der Form der Anordnung von Wärmequelle, Trägerelement und Wärmeleitelement zumindest in etwa folgenden und ist dabei insbesondere bogenförmig und/oder kugelförmig ausgebildet.

Bei einer bevorzugten Ausgestaltung ist zwischen dem Wärmeleitelement und dem Gehäuse ein Kühlelement angeordnet ist.

Diese Ausgestaltung ermöglicht es, einen höheren Wärmefluss von der Wärmequelle weg zu erzielen.

Bei einer bevorzugten Ausgestaltung ist das Kühlelement als Peltier-Element ausgebildet ist.

Diese Ausgestaltung ermöglicht es, einen nochmals höheren Wärmefluss von der Wärmequelle weg zu erzielen. Dabei ist das Peltier-Element mit seiner kühlenden Seite der Wärmequelle zugewandt und mit seiner wärmeabgebenden Seite dem Gehäuse. Die reduzierte Temperatur auf der Seite der Wärmequelle erhöht den Wärmefluss zum Peltier-Element. Die erhöhte Temperatur auf der Seite des Gehäuses erhöht den Wärmefluss vom Peltier-Element weg.

Bei einer bevorzugten Ausgestaltung weist die zweite Oberfläche einen ersten Oberflächenabschnitt und einen zweiten Oberflächenabschnitt auf, die in einem Winkel zueinander stehen.

Diese Ausgestaltung ermöglicht es, die Montierbarkeit und Fertigbarkeit zu vereinfachen.

Bei einer bevorzugten Ausgestaltung weist das Wärmesperrelement eines der Materialien ausgewählt aus der Gruppe bestehend aus Kunststoff, Edelstahl und Titan auf oder ist daraus gebildet.

Diese Materialien haben sich bei praktischen Versuchen als vorteilhaft für das Wärmesperrelement erwiesen.

Bei einer bevorzugten Ausgestaltung weist das Wärmeleitelement eines der Materialien ausgewählt aus der Gruppe bestehend aus Aluminium, Aluminium-Legierung, Kupfer und Kupfer-Legierung auf oder ist daraus gebildet.

Diese Materialien haben sich bei praktischen Versuchen als vorteilhaft für das Wärmeleitelement erwiesen.

Gemäß einem zweiten Aspekt wird die Aufgabe gelöst durch ein System nach Anspruch 11.

Bei dieser Ausgestaltung ist in dem System eine weitere, die zweite Wärmequelle vorhanden, die beispielsweise aus der Verlustwärme eines elektronischen Bauteils, zum Beispiel ein Field Programmable Gate Array (FPGA), oder aus Lichtverlusten resultiert. Dabei sei insbesondere angenommen, dass die zweite Wärmequelle eine höhere Temperatur als die erste Wärmequelle hat. Dies bedeutet, dass innerhalb des Gehäuses zusätzliche Wärme erzeugt wird, die die erste Wärmequelle, beispielsweise einen oder mehrere Bildsensoren, erwärmen könnte. Aufgrund des guten Wärmeflusses von der ersten Wärmequelle zum Gehäuse kann eine zusätzliche Erwärmung der ersten Wärmequelle verringert oder verhindert werden.

Bei einer bevorzugten Ausgestaltung ist die zweite Wärmequelle direkt am Gehäuse angeordnet.

Diese Anordnung bewirkt, dass ein erheblicher Teil der von der zweiten Wärmequelle abgegebenen Wärmeenergie direkt zum Gehäuse gelangt und von dort abgeführt wird, beispielsweise über die Luft.

Bei einer bevorzugten Ausgestaltung ist die zweite Wärmequelle mit einem Wärmeverteiler verbunden, der dafür ausgebildet ist, einen Wärmestrom von der Wärmequelle zum Gehäuse zu erhöhen.

Auf diese Weise kann ein verbleibender Wärmestrom von der zweiten Wärmequelle zur ersten Wärmequelle verringert werden. Dadurch kann eine zusätzliche Erwärmung der ersten Wärmequelle verringert oder verhindert werden. Außerdem bietet der Wärmeverteiler die Möglichkeit, eine entstehende Wärme auf eine größere Fläche zu verteilen, um so punktuell heiße Bereiche, auch hot spots genannt, an der Oberfläche zu vermeiden.

Bei einer bevorzugten Ausgestaltung weist der Wärmeverteiler einen Halter auf, an dem eine Heatpipe angeordnet ist, wobei die Heatpipe entlang des Gehäuses verläuft.

Diese Ausgestaltung erhöht den Wärmestrom von der zweiten Wärmequelle zum Gehäuse. Dadurch wird im Ergebnis ein verbleibender Wärmestrom von der zweiten Wärmequelle zur ersten Wärmequelle verringert. Eine zusätzliche Erwärmung der ersten Wärmequelle kann so verringert oder verhindert werden.

Ausführungsbeispiele der Erfindung sind in der Zeichnung dargestellt und werden in der nachfolgenden Beschreibung näher erläutert. Es zeigen:
- Fig. 1: eine Ausführungsform eines Systems mit einer Kühlvorrichtung;
- Fig. 2: einen ersten Schritt beim Zusammenbau einer Kühlvorrichtung gemäß einer ersten Ausführungsform;
- Fig. 3: einen zweiten Schritt des Aufbaus der ersten Ausführungsform der Kühlvorrichtung;
- Fig. 4: einen dritten Schritt des Aufbaus der ersten Ausführungsform der Kühlvorrichtung;
- Fig. 5: einen vierten Schritt des Aufbaus der ersten Ausführungsform der Kühlvorrichtung;
- Fig. 6: eine zweite Ausführungsform einer Kühlvorrichtung;
- Fig. 7: eine zweite Ausführungsform eines Systems mit einer Kühlvorrichtung;
- Fig. 8: eine dritte Ausführungsform eines Systems mit einer Kühlvorrichtung;
- Fig. 9: eine vierte Ausführungsform eines Systems mit einer Kühlvorrichtung;
- Fig. 10: eine vereinfachte Darstellung des Wärmeflusses von der zweiten Wärmequelle zum Gehäuse bei der zweiten Ausführungsform des Systems;
- Fig. 11: eine erste Ausführungsform einer Anordnung einer zweiten Wärmequelle relativ zum Gehäuse;
- Fig. 12: eine zweite Ausführungsform einer Anordnung einer zweiten Wärmequelle relativ zum Gehäuse;
- Fig. 13: eine dritte Ausführungsform einer Anordnung einer zweiten Wärmequelle relativ zum Gehäuse;
- Fig. 14: eine vierte Ausführungsform einer Anordnung einer zweiten Wärmequelle relativ zum Gehäuse; und
- Fig. 15: eine fünfte Ausführungsform einer Anordnung einer zweiten Wärmequelle relativ zum Gehäuse.

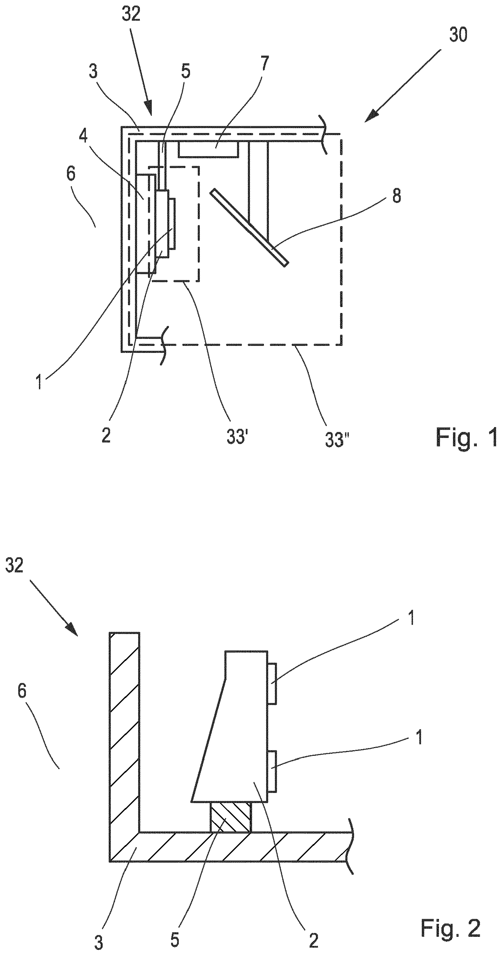

Fig. 1 zeigt ein System 30 mit einer Kühlvorrichtung 32, wobei innerhalb eines Gehäuses 3 eine erste Wärmequelle 1 und eine zweite Wärmequelle 7 angeordnet sind. Dabei ist es gewünscht, dass um die erste Wärmequelle 1 eine erste Temperaturzone 33' gebildet ist, wohingegen innerhalb des Gehäuses 3 eine zweite Temperaturzone 33" gebildet ist, deren Temperatur höher ist oder sein darf als die der ersten Temperaturzone 33'. Lediglich beispielhaft sind hier zudem optische Komponenten 8 innerhalb der zweiten Temperaturzone 33", aber außerhalb der ersten Temperaturzone 33', gezeigt.

Es wird nachfolgend nun genauer auf den Aufbau der Kühlvorrichtung 32 eingegangen.

Fig. 2 zeigt den ersten Schritt eines Zusammenbaus einer ersten Ausführungsform einer Kühlvorrichtung 32. Gezeigt sind hier zwei Wärmequellen 1, die hier jeweils durch einen Bildsensor oder eine LED gebildet werden. Die Wärmequellen 1 sind auf einem Trägerelement 2 angeordnet, wobei das Trägerelement 2 auf einem steifen Wärmesperrelement 5 angeordnet ist. Eine Umgebung 6 des Gehäuses 3 ist ebenfalls dargestellt.

Das Wärmesperrelement 5 ist dabei derart an dem Gehäuse 3 angeordnet, dass das Trägerelement 2, das Wärmesperrelement 5 und das Gehäuse 3 eine mechanisch steife Einheit bilden. Das Wärmesperrelement 5 hat eine geringe Wärmeleitfähigkeit. Dadurch ist eine stabile mechanische Anordnung der Wärmequellen 1 relativ zum Gehäuse 3 gewährleistet.

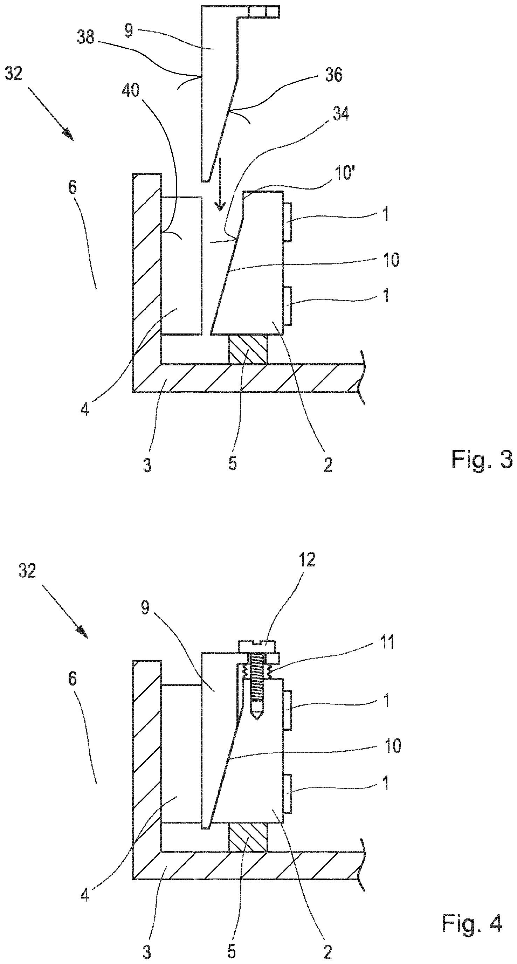

Fig. 3 zeigt einen zweiten Schritt des Zusammenbaus der Kühlvorrichtung 32. In der Figur sind eine erste Oberfläche 34 des Trägerelements 2, eine zweite Oberfläche 36 des Wärmeelements 9, eine dritte Oberfläche 38 des Wärmeelements 9 und eine vierte Oberfläche 40 des Gehäuses 3 gezeigt. Die erste Oberfläche 34 weist einen ersten Oberflächenabschnitt 10 und einen zweiten Oberflächenabschnitt 10' auf, die in einem Winkel zueinander stehen.

Es ist zudem zu erkennen, dass die zweite Oberfläche 36 in einem Winkel zur dritten Oberfläche 38 steht. Das Wärmeleitelement 9 ist separat von Trägerelement 2 und Gehäuse 3 ausgebildet. Das Wärmeleitelement 9 hat eine hohe Wärmeleitfähigkeit. Mittels des Pfeils ist angedeutet, dass das Wärmeleitelement 9 bei dem Zusammenbau zwischen das Trägerelement 2 und das Gehäuse 3 eingeschoben wird, hier zwischen das Trägerelement 2 und ein Kühlelement 4.

Das Wärmeleitelement 9 ist keilförmig ausgebildet. Ferner ist es dazu ausgebildet, zu seiner Positionierung zwischen das Trägerelement 2 und das Gehäuse 3 eingeschoben zu werden und dabei mit seiner zweiten Oberfläche 36 auf die erste Oberfläche 34 des Trägerelements aufzugleiten.

Fig. 4 zeigt einen dritten Schritt des Zusammenbaus der Kühlvorrichtung 32. Hier ist das Wärmeleitelement 9 nun zwischen das Trägerelement 2 und das Gehäuse 3, genauer zwischen das Trägerelement 2 und das Kühlelement 4, eingeschoben. In dieser Position liegt das Wärmeleitelement 9 an der ersten Oberfläche 34 des Trägerelements 2 mit seiner zweiten Oberfläche 36 an und ist mit seiner dritten Oberfläche 38 der vierten Oberfläche 40 des Gehäuses 3 zugewandt.

Für eine stabile Positionierung weist das Wärmeleitelement 9 ein Befestigungselement 12 auf, hier eine Schraube, mit dem das Wärmeleitelement 9 an dem Trägerelement 2 befestigt ist. Dabei weist das Befestigungselement 12 ein flexibles Element 11 auf, welches zwischen dem Wärmeleitelement 9 und dem Trägerelement 2 angeordnet ist. Das flexible Element 11 ist dafür ausgebildet, einen Druck zwischen dem Wärmeleitelement 9 und dem Trägerelement 2 zu verteilen und/oder zu reduzieren.

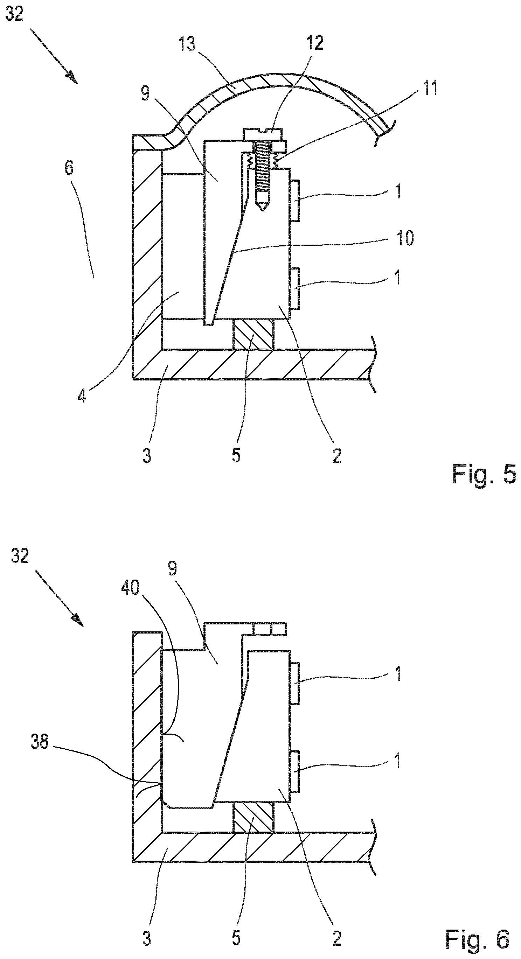

Fig. 5 zeigt einen vierten Schritt des Zusammenbaus der Kühlvorrichtung 32. Hier wurde nun ein Gehäusedeckel 13 angeordnet, der die Anordnung von Wärmequellen 1, Trägerelement 2 und Wärmeleitelement 9 überdeckt. Außerdem überdeckt der Gehäusedeckel 13 bei dieser Ausgestaltung auch das Kühlelement 4.

Fig. 6 zeigt eine zweite Ausführungsform der Kühlvorrichtung 32, bei der das Wärmeleitelement 9 mit seiner dritten Oberfläche 38 direkt an der vierten Oberfläche 40 anliegt.

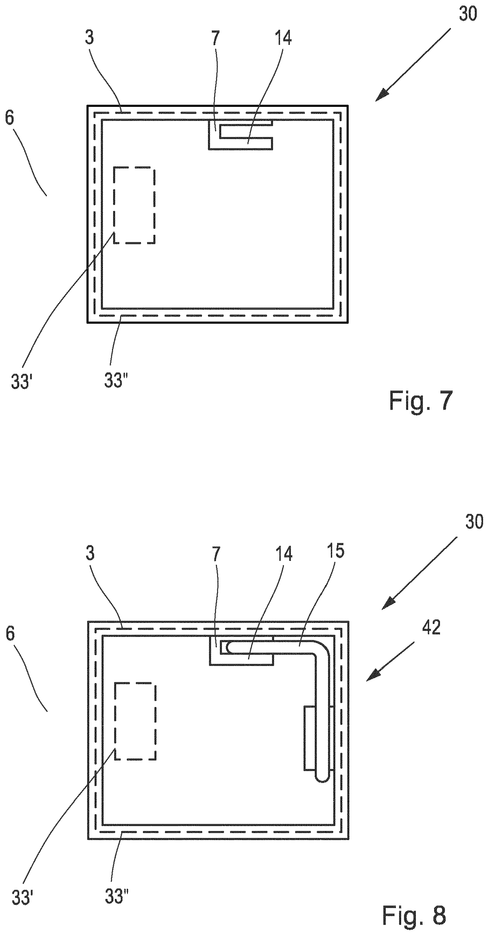

Fig. 7 zeigt eine symbolische Darstellung des Gehäuses 3 in einer Umgebung 6, wobei innerhalb des Gehäuses 3 die erste Temperaturzone 33' und die zweite Temperaturzone 33" gezeigt sind. Zudem ist erneut eine zweite Wärmequelle 7 gezeigt. Die zweite Wärmequelle 7 ist hier direkt am Gehäuse 3 angeordnet. An der zweiten Wärmequelle 7 ist ein Halter 14 eines Wärmeverteilers 42 angeordnet, der in der folgenden Figur gezeigt wird.

Fig. 8 zeigt, dass die zweite Wärmequelle 7 mit einem Wärmeverteiler 42 verbunden ist, der dafür ausgebildet ist, einen Wärmestrom von der zweiten Wärmequelle 7 zum Gehäuse 3 zu erhöhen. Der Wärmeverteiler 42 weist hier eine Heatpipe 15 auf, die entlang des Gehäuses 3 verläuft. Dabei verläuft die Heatpipe 15 insbesondere nahe einer Wand des Gehäuses 44 oder ist an einer Wand des Gehäuses 3 angeordnet. Durch die nun verbesserte Wärmeverteilung werden punktuell heiße Stellen an der Oberfläche vermieden.

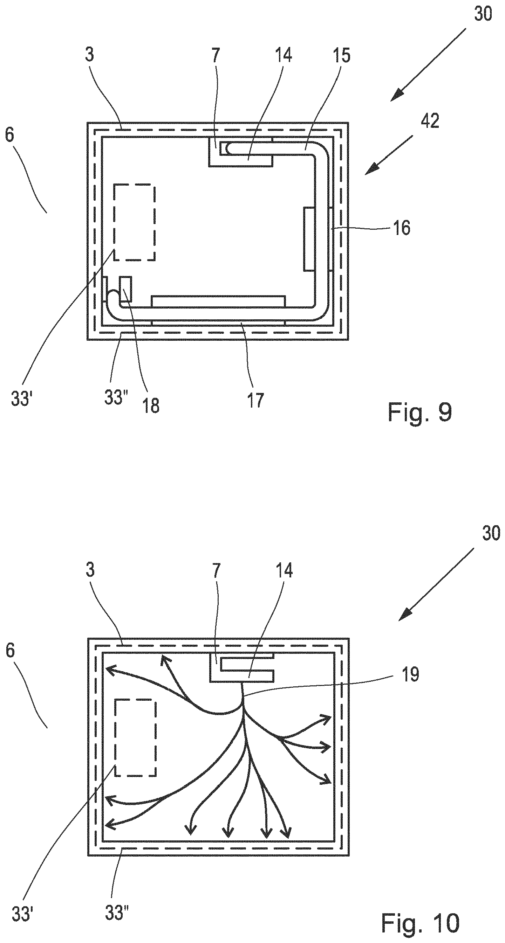

Fig. 9 zeigt eine dritte Ausführungsform eines Systems 30, wobei die Heatpipe 15 nun länger ist und in weiteren Haltern 16, 17, 18 geführt ist.

Fig. 10 zeigt symbolisch den Verlauf des Wärmestroms von der zweiten Wärmequelle 7 zum Gehäuse 3. Der Wärmestrom ist dabei mittels der Pfeile 19 symbolisiert.

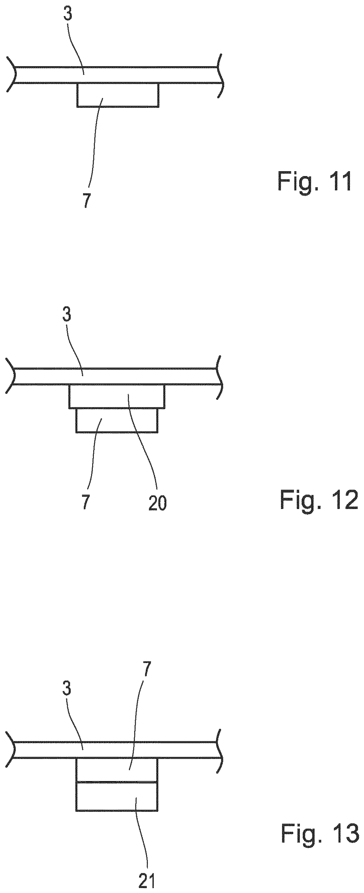

Fig. 11 zeigt eine erste Ausführungsform einer Anordnung einer zweiten Wärmequelle relativ zum Gehäuse. Hier ist die zweite Quelle 7 direkt am Gehäuse 3 angeordnet.

Fig. 12 zeigt eine zweite Ausführungsform einer Anordnung einer zweiten Wärmequelle relativ zum Gehäuse. Hier ist die zweite Wärmequelle 7 mittels eines Halters 20 am Gehäuse 3 befestigt, wobei der Halter 20 für eine Wärmeübertragung ausgebildet ist.

Fig. 13 zeigt eine dritte Ausführungsform einer Anordnung einer zweiten Wärmequelle relativ zum Gehäuse. Die zweite Wärmequelle 7 ist hier direkt am Gehäuse 3 angeordnet. Auf der zweiten Wärmequelle 7 ist ferner ein Kühler 21 angeordnet, der bevorzugt passiv ausgeführt ist.

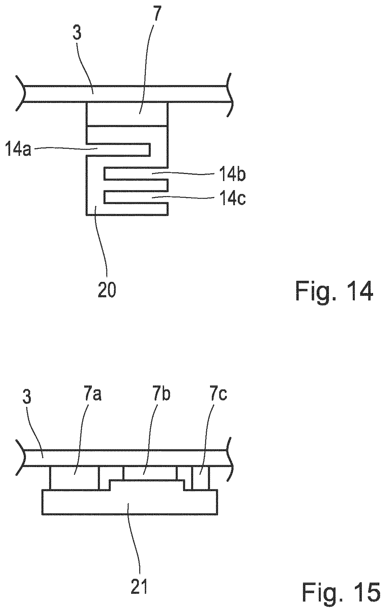

Fig. 14 zeigt eine vierte Ausführungsform einer Anordnung einer zweiten Wärmequelle relativ zum Gehäuse. Der Halter 20 ist hier derart ausgestaltet, dass er mehrere Ausnehmungen 14a, 14b, 14c aufweist, die mit Heatpipes verbunden werden können.

Fig. 15 zeigt eine fünfte Ausführungsform einer Anordnung einer zweiten Wärmequelle relativ zum Gehäuse. Bei dieser Ausführungsform leiten mehrere zweite Wärmequellen 7a, 7b, 7c einen Teil ihrer Wärme an einen gemeinsamen Kühler 21 ab.

Es sei darauf hingewiesen, dass die Wärmequelle insbesondere ein elektrisches oder elektronisches Bauteil ist oder aufweist, welches mit einer Betriebsspannung versorgt wird. Die Wärmequelle ist dabei beispielsweise ein Bildsensor, insbesondere ein CMOS-Bildsensor.

## Patentansprüche

1. Kühlvorrichtung (32) für ein Endoskop oder ein Exoskop, mit einer Wärmequelle (1), die auf einem Trägerelement (2) angeordnet ist, wobei das Trägerelement (2) auf einem steifen Wärmesperrelement (5) und das Wärmesperrelement (5) an einem Gehäuse (3) derart angeordnet ist, dass das Trägerelement (2), das Wärmesperrelement (5) und das Gehäuse (3) eine mechanisch steife Einheit bilden, wobei das Wärmesperrelement (5) eine geringe Wärmeleitfähigkeit hat, wobei ferner zwischen dem Gehäuse (3) und dem Trägerelement (2) ein Wärmeleitelement (9) angeordnet, das an einer ersten Oberfläche (34) des Trägerelements (2) mit einer zweiten Oberfläche (36) anliegt und mit einer dritten Oberfläche (38) einer vierten Oberfläche (40) des Gehäuses (3) zugewandt ist, wobei das Wärmeleitelement (9) eine hohe Wärmeleitfähigkeit hat, die zweite Oberfläche (36) in einem Winkel zur dritten Oberfläche (38) steht und das Wärmeleitelement (9) separat von Trägerelement (2) und Gehäuse (3) ausgebildet ist, **dadurch gekennzeichnet, dass** das Wärmeleitelement (9) dazu ausgebildet ist, zu seiner Positionierung zwischen das Trägerelement (2) und das Gehäuse (3) eingeschoben zu werden und dabei mit seiner zweiten Oberfläche (36) auf die erste Oberfläche (34) des Trägerelements (2) aufgleitet.

2. Kühlvorrichtung (32) nach Anspruch 1, wobei das Wärmeleitelement (9) keilförmig ausgebildet ist.

3. Kühlvorrichtung (32) nach einem der vorhergehenden Ansprüche, wobei das Wärmeleitelement (9) ein Befestigungselement (12) aufweist, mit dem das Wärmeleitelement (9) an dem Trägerelement (2) befestigt werden kann.

4. Kühlvorrichtung (32) nach Anspruch 3, wobei das Befestigungselement (12) ein flexibles Element (11) aufweist, welches zwischen dem Wärmeleitelement (9) und dem Trägerelement (2) angeordnet ist und dafür ausgebildet ist, einen Druck zwischen dem Wärmeleitelement (9) und dem Trägerelement (2) zu verteilen und/oder zu reduzieren.

5. Kühlvorrichtung (32) nach einem der vorhergehenden Ansprüche, wobei das Gehäuse (3) einen Gehäusedeckel (13) aufweist, der die Anordnung von Wärmequelle (1), Trägerelement (2) und Wärmeleitelement (9) überdeckt.

6. Kühlvorrichtung (32) nach einem der vorhergehenden Ansprüche, wobei zwischen dem Wärmeleitelement (9) und dem Gehäuse (3) ein Kühlelement (4) angeordnet ist.

7. Kühlvorrichtung (32) nach einem Anspruch 6, wobei das Kühlelement (4) als Peltier-Element ausgebildet ist.

8. Kühlvorrichtung (32) nach einem der vorhergehenden Ansprüche, wobei die erste Oberfläche (34) einen ersten Oberflächenabschnitt (10) und einen zweiten Oberflächenabschnitt (10') aufweist, die in einem Winkel zueinander stehen.

9. Kühlvorrichtung (32) nach einem der vorhergehenden Ansprüche, wobei das Wärmesperrelement (5) eines der Materialien ausgewählt aus der Gruppe bestehend aus Kunststoff, Edelstahl und Titan aufweist oder daraus gebildet ist.

10. Kühlvorrichtung (32) nach einem der vorhergehenden Ansprüche, wobei das Wärmeleitelement (9) eines der Materialien ausgewählt aus der Gruppe bestehend aus Aluminium, Aluminium-Legierung, Kupfer und Kupfer-Legierung aufweist oder daraus gebildet ist.

11. System mit einer Kühlvorrichtung (32) nach einem der vorhergehenden Ansprüche, wobei innerhalb des Gehäuses (3) zusätzlich zu der genannten, ersten Wärmequelle (1) eine zweite Wärmequelle (7) angeordnet ist.

12. System nach Anspruch 11, wobei die zweite Wärmequelle (7) direkt am Gehäuse (3) angeordnet ist.

13. System nach Anspruch 11 oder 12, wobei die zweite Wärmequelle (7) mit einem Wärmeverteiler (42) verbunden ist, der dafür ausgebildet ist, einen Wärmestrom von der zweiten Wärmequelle (7) zum Gehäuse (3) zu erhöhen.

14. System nach Anspruch 13, wobei der Wärmeverteiler (42) einen Halter (14) aufweist, an dem eine Heatpipe (15) angeordnet ist, wobei die Heatpipe (15) entlang des Gehäuses (3) verläuft.

## Claims

1. Cooling device (32) for an endoscope or an exoscope, comprising a heat source (1) arranged on a support element (2), wherein the support element (2) is arranged on a rigid heat barrier element (5) and the heat barrier element (5) is arranged on a housing (3) in such a way, that the support element (2), the heat barrier element (5) and the housing (3) form a mechanically rigid unit, the heat barrier element (5) having a low heat conductivity, further, a heat conducting element (9) being arranged between the housing (3) and the support element (2), which abuts a first surface (34) of the support element (2) with a second surface (36) and faces a fourth surface (40) of the housing (3) with a third surface (38), wherein the heat conducting element (9) has a high heat conductivity, the second surface (36) is at an angle to the third surface (38), and the heat conducting element (9) is formed separately from the support element (2) and the housing (3), **characterized in that** the heat conducting element (9) is configured to be inserted for its positioning between the support element (2) and the housing (3) and, when doing so, to slide with its second surface (36) onto the first surface (34) of the support element (2).

2. Cooling device (32) according to claim 1, wherein the heat conducting element (9) is wedge-shaped.

3. Cooling device (32) according to one of the preceding claims, wherein the heat conducting element (9) has a fastening element (12) with which the heat conducting element (9) can be fastened to the support element (2).

4. Cooling device (32) according to claim 3, wherein the fastening element (12) comprises a flexible member (11) which is arranged between the heat conducting element (9) and the support element (2) and is configured to distribute and/or reduce a pressure between the heat conducting element (9) and the support element (2).

5. Cooling device (32) according to one of the preceding claims, wherein the housing (3) has a housing cover (13) which covers the arrangement of heat source (1), support element (2) and heat conducting element (9).

6. Cooling device (32) according to one of the preceding claims, wherein a cooling element (4) is arranged between the heat conducting element (9) and the housing (3).

7. Cooling device (32) according to claim 6, wherein the cooling element (4) is configured as a Peltier element.

8. Cooling device (32) according to one of the preceding claims, wherein the first surface (34) has a first surface section (10) and a second surface section (10') which are at an angle to each other.

9. Cooling device (32) according to any of the preceding claims, wherein the heat barrier element (5) comprises or is formed of one of the materials selected from the group consisting of plastic, stainless steel and titanium.

10. Cooling device (32) according to any of the preceding claims, wherein the heat conducting element (9) comprises or is formed of one of the materials selected from the group consisting of aluminum, aluminum alloy, copper and copper alloy.

11. System with a cooling device (32) according to one of the preceding claims,
wherein a second heat source (7) is arranged within the housing (3) in addition to the previously mentioned first heat source (1).

12. System according to claim 11, wherein the second heat source (7) is arranged directly on the housing (3).

13. System according to claim 11 or 12, wherein the second heat source (7) is connected to a heat distributor (42) configured to increase a heat flow from the second heat source (7) to the housing (3).

14. System according to claim 13, wherein the heat distributor (42) comprises a holder (14) on which a heat pipe (15) is arranged, the heat pipe (15) extending along the housing (3).

## Revendications

1. Dispositif de refroidissement (32) pour un endoscope ou un exoscope, avec une source de chaleur (1) qui est disposée sur un élément de support (2), l'élément de support (2) étant disposé sur un élément de barrière thermique rigide (5) et l'élément de barrière thermique (5) étant disposé sur un boîtier (3) de telle manière, que l'élément de support (2), l'élément de barrière thermique (5) et le boîtier (3) forment une unité mécaniquement rigide, l'élément de barrière thermique (5) ayant une conductivité thermique faible, un élément conducteur de chaleur (9) étant en outre disposé entre le boîtier (3) et l'élément de support (2), qui est en contact avec une première surface (34) de l'élément de support (2) par une deuxième surface (36) et qui est tourné par une troisième surface (38) vers une quatrième surface (40) du boîtier (3), l'élément conducteur de chaleur (9) ayant une conductivité thermique élevée, la deuxième surface (36) formant un angle avec la troisième surface (38) et l'élément conducteur de chaleur (9) étant formé séparément de l'élément de support (2) et du boîtier (3), **caractérisé en ce que** l'élément conducteur de chaleur (9) est conçu pour être inséré entre l'élément de support (2) et le boîtier (3) pour son positionnement et glisse ainsi avec sa deuxième surface (36) sur la première surface (34) de l'élément de support (2).

2. Dispositif de refroidissement (32) selon la revendication 1, dans lequel l'élément conducteur thermique (9) est en forme de coin.

3. Dispositif de refroidissement (32) selon l'une des revendications précédentes, dans lequel l'élément conducteur de chaleur (9) comprend un élément de fixation (12) permettant de fixer l'élément conducteur de chaleur (9) à l'élément de support (2).

4. Dispositif de refroidissement (32) selon la revendication 3, dans lequel l'élément de fixation (12) comprend un élément flexible (11) qui est disposé entre l'élément conducteur de chaleur (9) et l'élément de support (2) et qui est adapté pour répartir et/ou réduire une pression entre l'élément conducteur de chaleur (9) et l'élément de support (2).

5. Dispositif de refroidissement (32) selon l'une des revendications précédentes, dans lequel le boîtier (3) comporte un couvercle de boîtier (13) qui recouvre l'agencement constitué par la source de chaleur (1), l'élément de support (2) et l'élément conducteur de chaleur (9).

6. Dispositif de refroidissement (32) selon l'une des revendications précédentes, dans lequel un élément conducteur de chaleur (4) est disposé entre l'élément conducteur de chaleur (9) et le boîtier (3).

7. Dispositif de refroidissement (32) selon l'une des revendications 6, dans lequel l'élément de refroidissement (4) est conçu comme un élément Peltier.

8. Dispositif de refroidissement (32) selon l'une des revendications précédentes, dans lequel la première surface (34) comprend une première partie de surface (10) et une deuxième partie de surface (10') qui sont disposées selon un angle l'une par rapport à l'autre.

9. Dispositif de refroidissement (32) selon l'une des revendications précédentes, dans lequel l'élément de barrière thermique (5) comprend ou est formé de l'un des matériaux sélectionnés parmi le groupe consistant en plastique, acier inoxydable et titane.

10. Dispositif de refroidissement (32) selon l'une des revendications précédentes, dans lequel l'élément conducteur de chaleur (9) comprend l'un des matériaux sélectionnés parmi le groupe consistant en l'aluminium, l'alliage d'aluminium, le cuivre et l'alliage de cuivre ou est formé à partir de ceux-ci.

11. Système comprenant un dispositif de refroidissement (32) selon l'une des revendications précédentes, dans lequel une deuxième source de chaleur (7) est disposée à l'intérieur du boîtier (3) en plus de ladite première source de chaleur (1).

12. Système selon la revendication 11, dans lequel la deuxième source de chaleur (7) est disposée directement sur le boîtier (3).

13. Système selon la revendication 11 ou 12, dans lequel la deuxième source de chaleur (7) est reliée à un distributeur de chaleur (42) configuré pour augmenter un flux de chaleur de la deuxième source de chaleur (7) vers le boîtier (3).

14. Système selon la revendication 13, dans lequel le distributeur de chaleur (42) comprend un support (14) sur lequel est disposé un caloduc (15), le caloduc (15) s'étendant le long du boîtier (3).
